(19) Europäisches Patentamt
European Patent Office
Office européen des brevets

(11) **EP 3 925 633 A1**

(12) **EUROPÄISCHE PATENTANMELDUNG**

(43) Veröffentlichungstag:
**22.12.2021 Patentblatt 2021/51**

(21) Anmeldenummer: **20181097.5**

(22) Anmeldetag: **19.06.2020**

(51) Int Cl.:
*A61L 9/20* (2006.01)          *C02F 1/32* (2006.01)
*F21V 7/00* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Virobuster International GmbH**
**53578 Windhagen (DE)**

(72) Erfinder: **Fehmi, Yigit**
**48599 Gronau (DE)**

(74) Vertreter: **Weidener, Jörg Michael**
**Von Rohr**
**Patentanwälte Partnerschaft mbB**
**Rüttenscheider Straße 62**
**45130 Essen (DE)**

(54) **BESTRAHLUNGSVORRICHTUNG ZUR UV-BESTRAHLUNG EINES STRÖMENDEN MEDIUMS**

(57) Die Erfindung betrifft eine Bestrahlungsvorrichtung (1) zur UV-Bestrahlung, insbesondere UV-C-Bestrahlung, eines die Bestrahlungsvorrichtung (1) durchströmenden Mediums, insbesondere Wasser oder Luft, insbesondere zur Inaktivierung von in dem Medium befindlichen Mikroorganismen, wie Bakterien, Keime, Schimmel und/oder Viren, mit einem einen Einlass (2) und einen Auslass (3) für das Medium aufweisenden zu durchströmenden Gehäuse (4) und wenigstens einer im Inneren des Gehäuses (4) angeordneten, UV-Strahlung emittierenden Strahlungsquelle (5) zur Bestrahlung des das Gehäuse (4) durchströmenden Mediums, wobei das Gehäuse (4) an der der Strahlungsquelle (5) zugewandten Innenseite (6) zumindest bereichsweise, vorzugsweise vollflächig, reflektierend mit einem Reflexionsgrad für die von der Strahlungsquelle (5) emittierten UV-Strahlung von wenigstens 0,6 ausgebildet ist, wobei die Strahlungsquelle (5) derart in dem Gehäuse (4) angeordnet ist, dass die von der Strahlungsquelle (5) emittierte Strahlung an der Innenseite (6) des Gehäuses (4) reflektiert wird und dass die von der Strahlungsquelle (5) emittierte Strahlung mit der reflektierten Strahlung konstruktiv interferiert.

Fig. 1

EP 3 925 633 A1

**Beschreibung**

Die Erfindung betrifft eine Bestrahlungsvorrichtung zur UV-Strahlung, insbesondere UV-C-Bestrahlung, eines die Bestrahlungsvorrichtung durchströmenden Mediums, insbesondere Wasser, Luft und/oder ein Gasgemisch und/oder ein Dampfgemisch, insbesondere zur Inaktivierung und/oder Abtötung von in dem Medium befindlichen Mikroorganismen, wie Bakterien, Keime, Schimmel und/oder Viren. Die Bestrahlungsvorrichtung weist einen Einlass und einen Auslass für das Medium bzw. den Mediumstrom auf, wobei der Einlass und der Auslass an einem von dem Medium durchströmten Gehäuse vorgesehen sind. Im Inneren des Gehäuses ist eine UV-Strahlung, insbesondere UV-C-Strahlung, emittierende Strahlungsquelle zur Bestrahlung des das Gehäuses durchströmenden Mediums angeordnet.

Insbesondere betrifft die vorliegende Erfindung das technische Gebiet der sogenannten UV-Desinfektion. Der Begriff "UV-Desinfektion" bezeichnet Verfahren, bei denen Mikroorganismen - auch als Mikroben bezeichnet - durch die Behandlung mit UV-Strahlung abgetötet werden können. Dabei kann die UV-Desinfektion zur Trinkwasseraufbereitung, zur Oberflächendesinfizierung, zur Abluftaufbereitung aber auch im Bereich der hygienischen Verarbeitung von Lebensmitteln oder dergleichen eingesetzt werden. Auch Luft in öffentlichen Bereichen kann so "sauber" - das heißt eine geringe Belastung mit schädlichen Mikroorganismen aufweisend - gehalten werden.

Als UV-C-Strahlung wird nachfolgend eine Strahlung mit einer Wellenlänge zwischen 100 nm bis 280 nm verstanden, insbesondere wobei im Rahmen der UV-Desinfektion UV-C-Strahlung bei einer Wellenlänge zwischen 200 nm bis 280 nm, vorzugsweise 240 nm bis 280 nm, eingesetzt wird.

Die UV-Desinfektion nutzt insbesondere eine Wellenlänge der UV-Strahlung zwischen 200 bis 300 nm. Dabei wirkt die abgegebene UV-Strahlung bakterizid - das heißt sie wird von der DNA absorbiert und zerstört deren Struktur und inaktiviert lebende Zellen. So können Mikroorganismen, wie Viren, Bakterien, Hefen und Pilze, mit UV-Strahlung innerhalb kürzester Zeit, insbesondere innerhalb von wenigen Sekunden, unschädlich gemacht werden.

Bei ausreichend hoher Bestrahlungsstärke ist die UV-Desinfektion somit eine zuverlässige und ökologische Methode, da insbesondere keine Zugabe von weiteren Chemikalien notwendig ist. Besonders vorteilhaft ist, dass Mikroorganismen keine Resistenz gegen UV-Strahlung entwickeln können. Letztlich kann durch die UV-Desinfektion auch die Vermehrung der Mikroorganismen unterbrochen werden.

Das Verfahren der UV-Desinfektion kann auch als "Ultraviolet Germicidal Irritation" (UVGI) und/oder als Mikroben-Desinfektion bezeichnet werden, insbesondere wobei UV-Strahlung mit einer Wellenlänge von 254 nm eingesetzt wird. Der Einsatz der UV-Desinfektion ist zur Virusinaktivierung bei der Luftreinigung besonders vorteilhaft, da so ermöglicht wird, große Luftvolumina von Viren zu befreien.

Der Ausbruch der Corona-Pandemie Anfang 2020 zeigt die dringende Notwendigkeit, wirtschaftliche, effiziente und ökologische Verfahren zur Virusinaktivierung vorzuhalten.

Ziel von Weiterentwicklungen ist es, Vorrichtungen bereitzustellen, die es ermöglichen, Medienströme mit einem großen Durchsatz, insbesondere Luftströme, effizient reinigen zu können. Ein weiteres oder alternatives Ziel ist es, auch diejenigen Partikel abzutöten, die vergleichsweise resistent gegen UV-Strahlung sind. Letztlich sind verschiedene Grundstrahlungsdosen bekannt, die in Abhängigkeit der zu tötenden Mikroorganismen voneinander abweichen können.

Die UV-Strahlungsdosis ($J/m^2$) ergibt sich aus der Multiplikation der UV-Bestrahlungsintensität ($W/m^2$) und der UV-Bestrahlungszeit (s).

Letztlich hängt die Inaktivierung der Mikroorganismen insbesondere von der Leistung oder der erreichten Intensität der UV-Strahlung ab. Auch die Distanz des Mediumstromes zur Strahlungsquelle hat einen Einfluss auf die Effizienz der Desinfektion bzw. Reinigung.

Aufgabe der vorliegenden Erfindung ist es, eine verbesserte Bestrahlungsvorrichtung zur UV-Bestrahlung, insbesondere UV-C-Bestrahlung, bereitzustellen, die bevorzugt eine effizientere Inaktivierung der Mikroorganismen ermöglicht.

Die vorgenannte Aufgabe wird erfindungsgemäß durch eine Bestrahlungsvorrichtung zur UV-Bestrahlung, insbesondere UV-C-Bestrahlung, eines die Bestrahlungsvorrichtung durchströmenden Mediums gelöst, wobei die Bestrahlungsvorrichtung ein einen Einlass und einen Auslass für das Medium aufweisendes Gehäuse, das von dem Medium durchströmt wird, aufweist und wenigstens eine im Inneren des Gehäuses angeordnete, UV-Strahlung emittierende Strahlungsquelle zur Bestrahlung des das Gehäuse durchströmenden Mediums. Das Gehäuse ist an der der Strahlungsquelle zugewandten Innenseite zumindest bereichsweise, vorzugsweise vollflächig, reflektierend mit einem Reflexionsgrad für die von der Strahlungsquelle emittierte UV-Strahlung von wenigstens 0,6 ausgebildet.

Die Strahlungsquelle ist erfindungsgemäße derart in dem Gehäuse angeordnet, dass die von der Strahlungsquelle emittierte Strahlung an der Innenseite des Gehäuses reflektiert wird und dass die von der Strahlungsquelle emittierte Strahlung mit der reflektierten Strahlung konstruktiv interferiert.

Alternativ oder zusätzlich ist erfindungsgemäß vorgesehen, dass die Strahlungsquelle derart in dem Gehäuse angeordnet ist, dass die von der Strahlungsquelle emittierte Strahlung an der Innenseite des Gehäuses reflektiert wird und dass die von der Strahlungsquelle emittierte Strahlung zu der reflektierten Strahlung einen von einem ganzzahligen Vielfachen der halben Wellenlänge und/oder von der halben Wellenlänge unterschiedlichen Gangunterschied aufweist. Ein Gangunterschied der insbesondere kohärenten Wellen bzw. Strahlungen der reflektierten Strahlung und der von der Strahlungsquelle emittierten Strahlung, der einem ganzzahligen Vielfachen der halben Wellenlänge und/oder der halben Wellenlänge entspricht, würde insbesondere eine destruktive Interferenz hervorrufen. Alternativ oder zusätzlich kann auch vorgesehen sein, dass die von der Strahlungsquelle emittierte Strahlung mit der reflektierten Strahlung zumindest im Wesentlichen nicht destruktiv interferiert, insbesondere wobei weniger als 30 %, bevorzugt weniger als 25 % und insbesondere zwischen 0 % bis 20 %, der von der Strahlungsquelle emittierten Strahlung destruktiv interferiert.

Erfindungsgemäß kann die Bestrahlungsvorrichtung zur Inaktivierung und/oder Abtötung von in dem Medium befindlichen Mikroorganismen, wie Bakterien, Keime, Schimmel und/oder Viren, eingesetzt werden. Als Medium kann Luft vorgesehen sein. Alternativ oder zusätzlich kann als Medium Wasser, Gas oder ein Dampfgemisch vorgesehen sein.

Insbesondere ist die Strahlungsquelle derart in dem Gehäuse angeordnet, dass die, bevorzugt von der Strahlungsquelle emittierte, Strahlung möglichst wenig gestört wird. Vorzugsweise wird eine geringe Störung und/oder nachteilige Beeinflussung der, bevorzugt von der Strahlungsquelle emittierten, Strahlung durch das Verhindern und/oder die Reduktion von dekonstruktiver Interferenz bzw. von dem Anteil an destruktiver Interferenz erreicht.

Ferner wird erfindungsgemäß die Strahlungsquelle insbesondere so angeordnet, dass die konstruktive Interferenz gezielt zur Erhöhung der Effizienz der Bestrahlungsvorrichtung eingesetzt wird. In diesem Zusammenhang sind unterschiedliche Ausführungen der erfindungsgemäßen Bestrahlungsvorrichtung denkbar, so dass ein auf konstruktiver Interferenz beruhendes Interferenzbild bzw. Interferenzmuster erreicht werden kann. Insbesondere kann entweder die Gehäusewand, insbesondere eine die Innenseite aufweisende Innenwand des Gehäuses, entsprechend ausgebildet sein und/oder die Strahlungsquelle kann relativ zu der Gehäuseinnenwand in einer bestimmten Art angeordnet sein.

Das Innere des Gehäuses bzw. der umschlossene Innenraum des Gehäuses kann insbesondere als UV-Behandlungskammer zur Behandlung des Mediums angesehen werden.

Bevorzugt ist die Innenwandung ein Reflektor, der weiter bevorzugt als in das Gehäuse eingeschobene und/oder austauschbare Gehäusekomponente ausgebildet ist.

Unter dem Reflexionsgrad wird im Sinne der Erfindung das Verhältnis zwischen reflektierter und einfallender Intensität als Energiegröße verstanden. Der Reflexionsgrad kann insbesondere in Abhängigkeit des Materials der Innenwandung, auf die die Strahlung auftrifft, und von der Strahlung selbst abhängen.

Interferenz beschreibt dabei die Änderung der Amplitude von zwei oder mehr Wellen nach dem Superpositionsprinzip. Interferenz tritt grundsätzlich bei unterschiedlichen Arten von Wellen auf, wobei im Rahmen der Erfindung die UV-Strahlung für die Interferenz relevant ist.

Destruktive Interferenz bezeichnet ein Phänomen, bei dem an einen bestimmten Ort die Wellen sich gegenseitig auslöschen. Dahingegen kennzeichnet die konstruktive Interferenz diejenigen Orte, wo sich die Wellen - sofern sie aufeinander treffen - verstärken. Die hat eine Erhöhung der Amplitude zur Folge.

Erfindungsgemäß wird insbesondere darauf abgezielt, die Amplitude der, insbesondere maximalen, Gesamt-Intensität der wechselwirkenden UV-Strahlung durch konstruktive Interferenz zu erhöhen. Im Inneren des Gehäuses entsteht somit ein durch konstruktive Interferenz geprägtes Interferenzmuster. In denjenigen Bereichen innerhalb des Gehäuses, wo die konstruktive Interferenz vorherrscht, kann eine besonders effiziente Abtötung der Viren ermöglicht werden.

Bisher ist in der Praxis die Anordnung der Strahlungsquelle innerhalb des Gehäuses lediglich von praktischen Überlegungen bestimmt gewesen. Wie in erfindungsgemäßen Versuchen festgestellt worden ist, ist bei in der Praxis bekannten Anordnungen der Strahlungsquelle innerhalb des Gehäuses vorgesehen, dass die destruktive Interferenz die konstruktive Interferenz übersteigt.

So ist es im Stand der Technik insbesondere bekannt, die Längsachse der Strahlungsquelle parallel und/oder koaxial zur Längsachse des Gehäuses anzuordnen. Zumeist ist die Strahlungsquelle zentral im Gehäuse angeordnet.

Die Längsachse des Gehäuses oder der Strahlungsquelle bezeichnet dabei insbesondere diejenige Achse, die in Richtung der längeren bzw. größten Ausdehnung des Körpers verläuft und somit die in Längsrichtung verlaufende Achse darstellt. Die Längsachse kann - muss jedoch nicht - eine annähernde Symmetrieachse des Gehäuses oder Strahlungsquelle sein.

Erfindungsgemäß gelingt es insbesondere, auch Bakterien, Viren und/oder Pilze mit einer vergleichsweise hohen UV-Resistenz abzutöten.

Vorzugsweise ist vorgesehen, dass die Strahlungsquelle derart in dem Gehäuse angeordnet ist, dass in dem durch Wechselwirkung der von der Strahlungsquelle emittierten Strahlung mit der reflektierten Strahlung erzeugten Interferenzbild der Anteil der konstruktiven Interferenz den Anteil der destruktiven Interferenz übersteigt, vorzugsweise um wenigstens 10 %, bevorzugt um wenigstens 20 %, weiter bevorzugt um wenigstens 40 %, vorzugsweise um wenigstens 50 %, insbesondere um wenigstens 70 %.

Alternativ oder zusätzlich kann vorgesehen sein, dass die Strahlungsquelle derart in dem Gehäuse angeordnet ist, dass der Anteil der wechselwirkenden und konstruktiv interferierenden Strahlung den Anteil der wechselwirkenden und destruktiv interferierenden Strahlung übersteigt, vorzugsweise um wenigstens 10 %, bevorzugt um wenigstens 20 %, weiter bevorzugt um wenigstens 40 %, vorzugsweise um wenigstens 50 %, insbesondere um wenigstens 70 %.

Letztlich wird insbesondere ein Interferenzbild dadurch erzeugt, dass die von der Strahlungsquelle emittierte Strahlung an der Innenseite des Gehäuses reflektiert wird und somit mit der emittierten Strahlung der Strahlungsquelle und der gesamten im Inneren des Gehäuses - bzw. in der UV-Behandlungskammer - befindlichen Strahlung wechselwirkt. In dem erfindungsgemäß erreichten Interferenzbild können die mit der konstruktiven Interferenz und/oder der geringen destruktiven Interferenz einhergehenden Vorteile gezielt genutzt werden, um die Effizienz der gesamten Vorrichtung zu verbessern.

Bei einer weiteren bevorzugten Ausführungsform ist vorgesehen, dass die Strahlungsquelle derart in dem Gehäuse angeordnet ist, dass das durch Wechselwirkung der von der Strahlungsquelle emittierten Strahlung mit der reflektierten Strahlung erzeugte Interferenzbild eine, insbesondere gemittelte, maximale Gesamt-Intensität des Interferenzbildes aufweist, die um wenigstens 50 %, bevorzugt wenigstens 100 %, weiter bevorzugt wenigstens 200 %, vorzugsweise wenigstens 280 %, weiter bevorzugt zwischen 300 % bis 500 %, größer als die maximale Intensität der direkten, von der Strahlungsquelle emittierten Strahlung vor - insbesondere zumindest im Wesentlichen unmittelbar - Reflektion an der Innenseite des Gehäuses ist. Insbesondere wird die Gesamt-Intensität durch die Überlagerung bzw. durch die konstruktive Interferenz der miteinander im Gehäuse wechselwirkenden Strahlung erreicht, so überlagert sich konstruktiv insbesondere die von der Strahlungsquelle emittierte Strahlung mit der reflektierten Strahlung. Auch die reflektierte Strahlung kann insbesondere mehrfach reflektiert werden, so dass sich insbesondere eine Mehrzahl von Inter-Reflektionen ergeben, die im Interferenzbild miteinander wechselwirken können.

Diese Erhöhung der Gesamt-Intensität ermöglicht insbesondere eine effiziente Abtötung der Mikroorganismen, insbesondere der Viren, da die Inaktivierung der Mikroben letztlich insbesondere zur Intensität der Strahlung korreliert, auf die die Mikroben treffen. Weiteren Einfluss hat diejenige Zeitdauer, in der die Mikroben der UV-Strahlung ausgesetzt sind.

Durch die Erhöhung der Intensität der UV-Strahlung kann somit die Strahlungsdosis erhöht werden, insbesondere bei der gleichen Zeitdauer der Aussetzung der Mikroorganismen der UV-C-Strahlung.

Zudem wird die Effizienz der UV-Desinfektion beeinflusst durch den Widerstandswert der Mikroorganismen ($k$ [$m^2$/J]) gegen die UV-Strahlung, die Aussetzungszeit ($t$ [s]) sowie durch die Intensität der Strahlung ($I$ [W/$m^2$]). Der Widerstandswert $k$ der Mikroorganismen kann nicht beeinflusst werden und kann insbesondere zwischen 0,001 bis 0,5 $m^2$/J, bevorzugt zwischen 0,0014 bis 0,3 $m^2$/J liegen.

Erfindungsgemäß kann erreicht werden, dass bei einer vorgegebenen UV-Resistenz (k-Faktor) eine geringe Aussetzungszeit durch die Erhöhung der Gesamt-Intensität ausreichend ist, da die Größen sich letztlich insbesondere gegenseitig beeinflussen. So kann sowohl durch eine Erhöhung der Aussetzungszeit als auch durch eine Erhöhung der Intensität die Inaktivierung der Mikroorganismen nach der Überwindung der UV-Resistenz erreicht werden.

Die Abtötungsrate der Mikroorganismen kann insbesondere nach folgender Formel bestimmt werden:

$$S\,(t) = S_0\,e^{-I \cdot k \cdot t}$$

mit:

$I$ = Intensität (W/$m^2$),
$k$ = UV-Rate des Mikroorganismus ($m^2$/J),
$t$ = die Verweilzeit (s)
$S$ = Mikrobengehalt
$S_o$ = Anfangsgehalt der Mikroben.

Bei einer weiteren ganz besonders bevorzugten Ausführungsform des Erfindungsgedankens ist vorgesehen, dass die Innenseite des Gehäuses ein Reflexionsgrad für die von der Strahlungsquelle emittierte UV-Strahlung von wenigstens 0,7, bevorzugt von wenigstens 0,8, weiter bevorzugt von wenigstens 0,9, aufweist. Insbesondere ist der Reflexionsgrad derart gewählt, dass die erfindungsgemäßen Interferenzen hervorgerufen werden können, die durch Wechselwirkung der an der Innenseite des Gehäuses reflektierten Strahlung mit der im Inneren des Gehäuses befindlichen Strahlung hervorgerufen werden können.

Besonders bevorzugt wird lediglich ein geringer Anteil der UV-Strahlung an der Innenwandung des Gehäuses transmittiert. Bevorzugt beträgt der transmittierte Anteil der UV-Strahlung weniger als 0,15, bevorzugt weniger als 0,1, weiter bevorzugt weniger als 0,05.

Die Innenwandung des Gehäuses und/oder die Innenseite des Gehäuses kann mit einer UV-Strahlung reflektierenden Beschichtung, die insbesondere den gewünschten Reflexionsgrad der Innenseite des Gehäuses sicherstellt, zumindest bereichsweise, vorzugsweise vollflächig bzw. vollständig, beschichtet sein. Alternativ oder zusätzlich kann vorgesehen sein, dass die Innenwandung und/oder die Innenseite des Gehäuses als Material Metall aufweist und/oder daraus besteht. Insbesondere kann die Innenwandung ein Blech, insbesondere ein Aluminium aufweisendes Blech und/oder ein verzinktes Blech und/oder ein Edelstahlblech, aufweisen und/oder daraus bestehen. Dabei kann - wie zuvor erläutert - die Innenwandung des Gehäuses als in das Gehäuse einsetzbare Komponente ausgebildet sein. Besonders bevorzugt ist ein mehrmals beschichtetes Aluminiumblech als Material für die Innenwandung des Gehäuses vorgesehen.

Darüber hinaus ist bei einer weiteren bevorzugten Ausführungsform vorgesehen, dass die Innenwandung und/oder die Innenseite des Gehäuses zylinderförmig und/oder gewellt oder eben - insbesondere flach bzw. ohne Erhebungen - ausgebildet ist. Alternativ oder zusätzlich kann vorgesehen sein, dass die Innenwandung und/oder die Innenseite des Gehäuses eine Mehrzahl von Erhebungen und/oder Vertiefungen aufweist und/oder das die Innenwandung des Gehäuses rotationssymmetrisch ausgebildet ist. In weiteren Ausführungsformen kann die Innenwandung des Gehäuses auch nicht rotationssymmetrisch ausgebildet sein.

Letztlich kann die Innenwandung und/oder die Innenseite des Gehäuses derart ausgebildet werden, dass eine relative Anordnung der Strahlungsquelle zur Innenwandung und/oder Innenseite gewährleistet werden kann, bei der ein möglichst hoher Anteil an konstruktiver Interferenz am gesamten Interferenzbild in der UV-Behandlungskammer sichergestellt werden kann.

Bei einer weiteren bevorzugten Ausführungsform ist die Längsachse des Gehäuses, insbesondere die Längsachse des Innenraums bzw. der Behandlungskammer des Gehäuses und/oder der Innenwandung des Gehäuses, nicht koaxial zur Längsachse der Strahlungsquelle ausgerichtet. Besonders bevorzugt ist die Strahlungsquelle insbesondere nicht symmetrisch im Innenraum des Gehäuses angeordnet.

Bei einer weiteren ganz besonders bevorzugten Ausführungsform ist die Längsachse des Gehäuses, insbesondere die Längsachse des Innenraums bzw. der Behandlungskammer des Gehäuses und/oder der Innenwandung des Gehäuses, versetzt, vorzugsweise schräg, zu der Längsachse der Strahlungsquelle ausgerichtet. Insbesondere ist die Längsachse des Gehäuses, insbesondere des Innenraums des Gehäuses und/oder der Innenwandung des Gehäuses, mit einem Winkel $\alpha$ größer als 2°, bevorzugt zwischen 2° bis 50°, weiter bevorzugt zwischen 3° bis 15°, zu der Längsachse der Strahlungsquelle ausgerichtet bzw. schließt einen Winkel $\alpha$ in der vorgenannten Größenordnung zu der Längsachse der Strahlungsquelle ein.

Vorzugsweise ist die Strahlungsquelle dezentral - insbesondere nicht-mittig - in dem und/oder mit Bezug zu dem Gehäuse angeordnet. Durch die dezentrale Anordnung können die konstruktiven Interferenzeffekte hervorgerufen werden, da eine Positionierung der Strahlungsquelle insbesondere derart vorgenommen wird, dass die reflektierte Strahlung mit der von der Strahlungsquelle emittierten Strahlung konstruktiv interferiert.

Ferner ist vorzugsweise die wenigstens eine Strahlungsquelle mittels einer Halteeinrichtung im Innenraum des Gehäuses an dem Gehäuse befestigt. Die Halteeinrichtung kann insbesondere eine Mehrzahl von Streben zur Befestigung der Stirnenden der Strahlungsquelle aufweisen. Auch weitere Anordnungsmöglichkeiten der Strahlungsquelle innerhalb des Gehäuses sind denkbar. Letztlich ist die Halteeinrichtung bevorzugt derart ausgebildet, dass das durch die Wechselwirkung zwischen der von der Strahlungsquelle emittierten Strahlung und/oder der, insbesondere "alten", reflektierten Strahlung, bevorzugt der zuvor reflektierten Strahlung, und der an der Gehäuseinnenwand, insbesondere nun neu, reflektierten Strahlung gebildete Interferenzbild möglichst wenig gestört wird.

Vorzugsweise wird diese geringe Störung bzw. geringe nachteilige Beeinflussung durch das Verhindern und/oder die Reduktion von dekonstruktiver Interferenz ermöglicht, insbesondere wobei die Strahlen und/oder die verschiedenen Anteile der Strahlung sich einander zumindest im Wesentlichen nicht auslöschen (destruktive Interferenz) können und/oder der Anteil an ausgelöschter Strahlung bzw. ausgelöschten Strahlen stark reduziert werden kann. Der Effekt, dass die Strahlung möglichst wenig ausgelöscht wird, wird insbesondere aufgrund fehlender Symmetrie bzw. fehlender symmetrischer Anordnung der Strahlungsquelle in dem Gehäuse erreicht.

Die dezentrale und/oder nicht-symmetrische und/oder schräge Anordnung der Strahlungsquelle innerhalb des Gehäuses kann gewährleisten, dass die Wellenlängen der Strahlen und/oder der verschiedenen Anteile der aufeinander treffenden Strahlung nicht versetzt um eine halbe Periode bzw. nicht versetzt um ein Vielfaches der halben Wellenlänge aufeinander treffen können. Eine solche Versetzung - die auch als Gangunterschied bezeichnet werden kann - würde andernfalls eine destruktive Interferenz hervorrufen.

Alternativ oder zusätzlich kann die Halteeinrichtung auch zumindest bereichsweise reflektierend ausgebildet sein und somit insbesondere zur erfindungsgemäßen Erhöhung der konstruktiven Interferenz beitragen. Besonders bevorzugt kann die Halteeinrichtung die stirnseitigen Enden der Strahlungsquelle mit wenigstens zwei, bevorzugt wenigstens drei, Haltemitteln, die insbesondere strebenartig ausgebildet sind und/oder an der Innenwandung des Gehäuses befestigt sind, halten. Vorzugsweise wird wenigstens ein Stirnende der Strahlungsquelle mit wenigstens drei strebenförmigen Haltemitteln mit der Innenwandung des Gehäuses befestigt. Somit kann letztlich die Strahlungsquelle innerhalb des Gehäuses gelagert werden. Bevorzugt kann durch die Halteeinrichtung die relative Ausrichtung der Strahlungsquelle zu der Gehäuseinnenwand vorgegeben werden.

Vorzugsweise ist die Halteeinrichtung zur Verstellung der Ausrichtung der Strahlungsquelle ausgebildet. Durch die Verstellbarkeit der Halteeinrichtung kann des Weiteren ermöglicht werden, dass insbesondere diejenige Stellung der Strahlungsquelle gefunden wird, die einen möglichst hohen Anteil an konstruktiver Interferenz ermöglicht.

Vorzugsweise weist das Gehäuse eine Länge zwischen 30 bis 200 cm, bevorzugt zwischen 80 bis 150 cm, auf. Das Gehäuse kann einen Durchmesser, vorzugsweise einen Innendurchmesser, zwischen 8 bis 30 cm, bevorzugt zwischen 10 bis 20 cm, aufweisen. Bei einer besonders bevorzugten Ausführungsform weist das Gehäuse eine Länge zwischen 80 bis 150 cm und einen Innendurchmesser zwischen 10 bis 20 cm auf. Bei den vorgenannten Größenordnungen kann insbesondere für einen Mediumstrom mit einer Strömungsgeschwindigkeit zwischen 1 bis 2 m/s eine effiziente Inaktivierung der Mikroorganismen gewährleistet werden.

Darüber hinaus kann die erfindungsgemäße Bestrahlungseinrichtung auch in Klimaanlagen oder Bürogebäuden oder dergleichen eingesetzt werden. Dabei versteht es sich, dass die Bestrahlungsvorrichtung in Abhängigkeit des Mediumstromes und/oder des Durchsatzes des Mediumstromes ausgebildet sein kann.

Ferner kann bei einer weiteren bevorzugten Ausführungsform vorgesehen sein, dass die Strahlungsquelle eine Mehrzahl von Leuchtmitteln, vorzugsweise LEDs, aufweist. Darüber hinaus kann alternativ oder zusätzlich die Strahlungsquelle eine zumindest im Wesentlichen langgestreckte und/oder stabförmige Form aufweisen. Insbesondere kann die Strahlungsquelle eine Länge von wenigstens 5 cm, bevorzugt zwischen 5 cm bis 30 cm, weiter bevorzugt zwischen 10 cm bis 20 cm, aufweisen. Bei einer besonders bevorzugten Ausführungsform ist vorgesehen, dass die Strahlungsquelle eine langgestreckten Form mit einer Mehrzahl von Leuchtmitteln, vorzugsweise LEDs aufweist, die entlang eines "arrays" angeordnet sind.

Zudem kann die Strahlungsquelle einen Durchmesser von wenigstens 1 cm, bevorzugt zwischen 1 cm und 20 cm, weiter bevorzugt zwischen 2 cm und 10 cm und insbesondere von 5 cm +/- 1 cm, aufweisen. Der Durchmesser der Strahlungsquelle kann insbesondere auch von dem Durchmesser des in der Strahlungsquelle eingesetzten Leuchtmittels abhängen.

Alternativ oder zusätzlich kann die Strahlungsquelle auch als UV-Niederdrucklampen, insbesondere eine Niederdruck-Quecksilber-Entladungslampe, und/oder als UV-Mitteldrucklampe ausgebildet sein.

Letztlich kann die Strahlungsquelle die zur Inaktivierung der Mikroorganismen benötigte UV-Strahlung, insbesondere die UV-C-Strahlung, bereitstellen. Dabei kann die Strahlungsquelle an ihrer Oberfläche eine Intensität zwischen 1000 bis 8000 W/m$^2$, bevorzugt zwischen 2000 bis 6000 W/m$^2$ und insbesondere von 4200 W/m$^2$ +/- 20 %, aufweisen.

Des Weiteren kann die Strahlungsquelle insbesondere eine Leistung von wenigstens 100 W, bevorzugt von 190 W +/- 10 %, erbringen. Die Leistung im Bereich der UV-C-Strahlung kann bevorzugt zwischen 10 bis 100 W, weiter bevorzugt zwischen 50 bis 70 W, betragen. Die von der Strahlungsquelle emittierte Strahlung kann mit ihrer Intensität im Abstandsquadrat abnehmen. Durch die erreichte konstruktive Interferenz kann dieser Reduzierung der Amplitude entgegengewirkt werden.

Bei einer weiteren bevorzugten Ausführungsform des Erfindungsgedankens ist vorgesehen, dass eine Mehrzahl von Strahlungsquellen in dem Gehäuse angeordnet ist. Insbesondere können in dem Gehäuse zwischen 2 bis 10, bevorzugt zwischen 2 bis 5, Strahlungsquellen angeordnet sein. Die Strahlungsquellen können jeweils insbesondere ebenfalls eine Mehrzahl von Leuchtmitteln, vorzugsweise LEDs, aufweisen. Durch die mehreren Strahlungsquellen kann über die Länge des Gehäuses eine zumindest im Wesentlichen gleichmäßige und/oder zur Inaktivierung der Mikroorganismen ausreichende Strahlung bereitgestellt werden.

Die Strahlung der mehreren Strahlungsquellen kann des Weiteren auch in dem entstehenden Interferenzbild in der UV-Behandlungskammer miteinander wechselwirken und zur Erhöhung der konstruktiven Interferenz beitragen. Die erfindungsgemäß angegebene Eignung zur konstruktiven Interferenz gilt insbesondere für jede Strahlungsquelle, die in der erfindungsgemäßen Bestrahlungsvorrichtung eingesetzt wird.

Grundsätzlich ist es auch möglich, dass die Strahlungsquellen voneinander unterschiedlich ausgebildet sind. Bevorzugt wird eine zumindest im Wesentlichen baugleiche Ausbildung der Strahlungsquellen vorgesehen.

Vorzugsweise sind die mehreren Strahlungsquellen zumindest bereichsweise überlappend in dem Gehäuse angeordnet. Die Länge des Überlappungsbereiches von wenigstens zwei Strahlungsquellen kann wenigstens 20 %, bevorzugt wenigstens 30 %, weiter bevorzugt zwischen 30 % bis 80 %, der Länge wenigstens einer Strahlungsquelle entsprechen. Der Überlappungsbereich kann insbesondere in Abhängigkeit der Länge des Gehäuses und/oder der Breite des Gehäuses und/oder in Abhängigkeit der unterschiedlichen Strahlungsquellen gewählt werden. Zudem ist beim Zustandekommen der Erfindung festgestellt worden, dass der Überlappungsbereich der Strahlungsquellen insbesondere nicht die erfindungsgemäße konstruktive Interferenz der Strahlung besonders nachteilig beeinflusst. So kann letztlich die Leistung der UV-Strahlung innerhalb des Gehäuses durch die mehreren Strahlungsquellen erhöht werden. Dies trägt ebenfalls zu einer effizienteren bzw. verbesserten Abtötung der Mikroben bei.

Bevorzugt können die mehreren Strahlungsquellen, insbesondere wenigstens zwei Strahlungsquellen, zueinander windschief und/oder schräg angeordnet sein. In einer alternativen oder zusätzlichen Ausführungsform kann auch vorgesehen sein, dass die Längsachsen der Strahlungsquellen zumindest im Wesentlichen parallel zueinander ausgerichtet sind.

Besonders bevorzugt sind die Längsachsen der Strahlungsquellen zueinander versetzt, vorzugsweise schräg, angeordnet. Die Längsachsen der Strahlungsquellen können einen Winkel β zwischen 1° bis 90°, bevorzugt zwischen 2° bis 50°, weiter bevorzugt zwischen 10° bis 30°, zueinander einschließen. Der Winkel β kann insbesondere in Abhängigkeit des hervorgerufenen Interferenzbildes innerhalb des Gehäuses gewählt werden, wobei im Interferenzbild eine möglichst hoher Anteil der konstruktiven Interferenz erreicht werden kann.

Bei einer weiteren bevorzugten Ausgestaltung ist vorgesehen, dass die Vorrichtung einen Ventilator zum Erzeugen des Mediumstromes vom Einlass zum Auslass umfasst. Der Ventilator ist insbesondere derart eingestellt, dass der Mediumstrom eine Durchströmungsgeschwindigkeit von 1 bis 5 m/s, bevorzugt zwischen 1 bis 2 m/s, aufweisen kann. Die Durchströmungsgeschwindigkeit kann insbesondere auch von dem Innendurchmesser des Gehäuses und/oder von der erreichten konstruktiven Interferenz in der UV-Behandlungskammer (das heißt im Innenraum des Gehäuses) abhängen.

Bevorzugt ist der Ventilator stromaufwärts zu der UV-Behandlungskammer bzw. zu der Strahlungsquelle und/oder dem Innenraum des Gehäuses angeordnet, um den Mediumstrom vom Einlass zu dem Auslass zu erzeugen.

Der Mediumstrom kann zumindest im Wesentlichen laminar durch den Innenraum des Gehäuses geführt werden. Alternativ oder zusätzlich kann aber auch vorgesehen sein, dass der in dem Gehäuse befindliche bzw. das Gehäuse durchströmende Mediumstrom eine turbulente Strömung ausbildet.

Des Weiteren kann mindestens eine Strahlungsquelle UV-Strahlung in einem Wellenlängenbereich von wenigstens 240 bis 300 nm, bevorzugt in einem Wellenlängenbereich von 250 bis 285 nm, weiter bevorzugt von 270 bis 280 nm und insbesondere von 254 nm +/- 10 % und/oder von 278 nm +/- 10 %, emittieren. Bei einer UV-C-Strahlung mit einer Wellenlänge von 254 nm +/- 10 % kann insbesondere eine hohe Vireninaktivierung erreicht werden. UV-Strahlung mit einer Wellenlänge in der vorgenannten Größenordnung ermöglicht erfindungsgemäß die Abtötung der Mikroorganismen in dem Mediumstrom.

Vorzugsweise ist vor dem Einlass ein Vorfilter angeordnet. Der Vorfiter kann bevorzugt derart ausgebildet sein, dass Partikel mit einem Durchmesser größer 1 μm, bevorzugt größer 0,5 μm, aus dem Mediumstrom herausgefiltert werden. Somit können insbesondere aus dem Mediumstrom solche Partikel herausgefiltert werden, die andernfalls eine sogenannte "Schattenbildung" in der UV-Behandlungskammer bei der entstehenden Wechselwirkung mit der UV-Strahlung erzeugen würden. Letztlich ist in diesem Zusammenhang relevant, dass die UV-Strahlung in einer Größenordnung zwischen 0,2 bis 0,3 μm liegt. Da die vorgenannten Partikel, beispielsweise Staubpartikel oder Pollen oder dergleichen, jedoch einen größeren Durchmesser als die Wellenlänge aufweisen, kann die Wellenlänge insbesondere nicht die Partikel mit einem Durchmesser von größer als 1 μm passieren. Ein Bakterium kann beispielsweise einen Durchmesser von circa 0,3 μm aufweisen. Erfindungsgemäß ist festgestellt worden, dass der Schatteneffekt vor Partikeln mit einem Durchmesser unter 0,5 μm, insbesondere zwischen 0,3 μm bis 0,5 μm, zwar vorhanden ist, jedoch noch die erreichte Abtötung der Mikroorganismen toleriert werden kann. So können auch diejenigen Mikroorganismen mit einem Durchmesser größer als die Wellenlänge der UV-Strahlung ebenfalls unschädlich gemacht werden, da auch auf sie die UV-Strahlung trifft.

Im Übrigen versteht es sich, dass in den vorgenannten Intervallen und Bereichsgrenzen jegliche Zwischenintervalle und Einzelwerte enthalten und als erfindungswesentlich offenbart anzusehen sind, auch wenn diese Zwischenintervalle und Einzelwerte nicht konkret angegeben sind.

Weitere Merkmale, Vorteile und Anwendungsmöglichkeiten der vorliegenden Erfindung ergeben sich aus der nachfolgenden Beschreibung von Ausführungsbeispielen anhand der Zeichnung und der Zeichnung selbst. Dabei bilden alle beschriebenen und/oder bildlich dargestellten Merkmale für sich oder in beliebiger Kombination den Gegenstand der vorliegenden Erfindung, unabhängig von ihrer Zusammenfassung in den Ansprüchen oder deren Rückbeziehung.

Es zeigt:

Fig. 1     eine schematische perspektivische Darstellung einer erfindungsgemäßen Bestrahlungsvorrichtung,

Fig. 2     eine schematische Vorderansicht der in Fig. 1 dargestellten Bestrahlungsvorrichtung,

Fig. 3     eine schematische Draufsicht auf die in Fig. 1 dargestellte Bestrahlungsvorrichtung,

Fig. 4     eine schematische Seitenansicht der in Fig. 1 dargestellten Bestrahlungsvorrichtung,

Fig. 5     eine schematische perspektivische Darstellung einer weiteren Ausführungsform der erfindungsgemäßen Bestrahlungsvorrichtung,

Fig. 6     eine schematische perspektivische Darstellung einer weiteren Ausführungsform der erfindungsgemäßen Bestrahlungsvorrichtung,

Fig. 7     eine schematische perspektivische Darstellung einer weiteren Ausführungsform der erfindungsgemäßen Bestrahlungsvorrichtung und

Fig. 8    eine schematische perspektivische Darstellung eines erfindungsgemäßen Gehäuses.

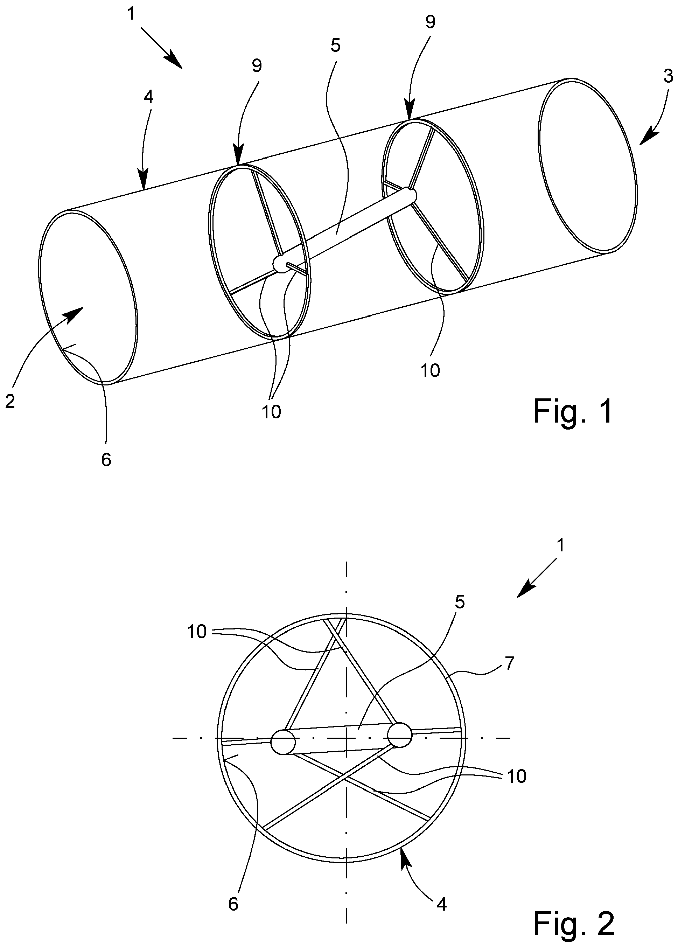

Fig. 1 zeigt eine Bestrahlungsvorrichtung 1 zur UV-Strahlung, insbesondere zur UV-C-Bestrahlung, eines die Bestrahlungsvorrichtung 1 durchströmenden Mediums. Als Medium kann Wasser oder Luft vorgesehen sein. In weiteren Ausführungsformen kann als Medium auch ein Gasgemisch und/oder ein Dampfgemisch verwendet werden.

Die Bestrahlungsvorrichtung 1 wird insbesondere zur Inaktivierung bzw. Abtötung von in dem Medium befindlichen Mikroorganismen, wie Bakterien, Keime, Schimmel und/oder Viren, eingesetzt.

Die Bestrahlungsvorrichtung 1 weist einen Einlass 2 und einen Auslass 3 auf. Der Einlass 2 dient zum Einlass des Mediums und der Auslass 3 zum Auslass des Mediums aus der Bestrahlungsvorrichtung 1. Die Bestrahlungsvorrichtung 1 umfasst ferner ein Gehäuse 4, das den Einlass 2 und den Auslass 3 aufweist und das von dem Medium durchströmt wird. In dem Gehäuse 4 ist wenigstens eine Strahlungsquelle 5 angeordnet. Die Strahlungsquelle 5 kann mit dem Gehäuse 4 verbunden und/oder befestigt und/oder an dem Gehäuse 4 gelagert sein. Fig. 1 zeigt, dass die zwei Stirnseiten der Strahlungsquelle 5 an dem Gehäuse 4 befestigt sind.

Das Gehäuse 4 ist an der der Strahlungsquelle 5 zugewandten Innenseite 6 zumindest bereichsweise, vorzugsweise vollflächig, reflektierend mit einem Reflexionsgrad für die von der Strahlungsquelle 5 emittierte UV-Strahlung, insbesondere der UV-C-Strahlung, von wenigstens 0,6 ausgebildet. In dem in Fig. 1 dargestellten Ausführungsbeispiel ist vorgesehen, dass der Reflexionsgrad wenigstens 0,7, bevorzugt wenigstens 0,8, beträgt.

Die Strahlungsquelle 5 ist derart in dem Gehäuse 4 angeordnet, dass die von der Strahlungsquelle 5 emittierte Strahlung an der Innenseite 6 des Gehäuses 4 reflektiert wird und dass die von der Strahlungsquelle 5 emittierte Strahlung mit der reflektierten Strahlung konstruktiv interferiert.

In weiteren Ausführungsformen kann die Strahlungsquelle 5 derart in dem Gehäuse 4 angeordnet ist, dass die von der Strahlungsquelle 5 emittierte Strahlung an der Innenseite 6 des Gehäuses 4 reflektiert wird und dass die von der Strahlungsquelle 5 emittierte Strahlung zu der reflektierten Strahlung einen von einem ganzzahligen Vielfachen der halben Wellenlänge und/oder von der halben Wellenlänge unterschiedlichen Gangunterschied aufweist und/oder dass die von der Strahlungsquelle 5 emittierte Strahlung mit der reflektierten Strahlung zumindest im Wesentlichen nicht destruktiv interferiert, insbesondere wobei weniger als 30 %, bevorzugt weniger als 25 % und insbesondere zwischen 0 % bis 20 %, der von der Strahlungsquelle 5 emittierten Strahlung destruktiv interferiert.

Die Innenseite 6 des Gehäuses 4 kann auch Bestandteil einer Innenwandung 7 sein. Die Innenwandung 7 kann in weiteren, nicht dargestellten Ausführungsbeispielen als austauschbare Komponente bzw. als aus dem Gehäuse 4 entnehmbare Komponente ausgebildet sein. Die Innenwandung 7 und/oder die Innenseite 6 ist im dargestellten Ausführungsbeispiel ein Reflektor, der zur Reflektion der von der Strahlungsquelle 5 ausgesendeten Strahlung ausgebildet ist.

Im Innenraum des Gehäuses 4 bzw. in der im Inneren gebildeten Behandlungskammer 8 kann somit ein Interferenzbild bzw. ein Interferenzmuster erzeugt werden, das durch konstruktive Interferenz charakterisiert wird. Insbesondere ist die Strahlungsquelle 5 derart in dem Gehäuse 4 angeordnet, dass die konstruktive Interferenz die destruktive Interferenz übersteigt, so dass die vorteilhaften Eigenschaften der konstruktiven Interferenz genutzt werden können. Vorzugsweise übersteigt die konstruktive Interferenz die destruktive Interferenz um wenigstens 10 %, bevorzugt um wenigstens 50 %.

Letztlich kann die Strahlungsquelle 5 derart in dem Gehäuse 4 angeordnet sein, dass der Anteil der wechselwirkenden und konstruktiv interferierenden Strahlung den Anteil der wechselwirkenden und destruktiv interferierenden Strahlung übersteigt, vorzugsweise um wenigstens 10 %, bevorzugt um wenigstens 20 %, weiter bevorzugt um wenigstens 50 %.

Fig. 1 zeigt, dass die Strahlungsquelle 5 derart in dem Gehäuse 4 angeordnet ist, dass das durch Wechselwirkung der von der Strahlungsquelle 5 emittierten Strahlung mit der reflektierten Strahlung erzeugte Interferenzbild eine, insbesondere gemittelte, maximale Gesamt-Intensität des Interferenzbildes aufweist, die um wenigstens 50 %, bevorzugt zwischen 300 % bis 500 %, größer als die maximale Intensität der direkten, von der Strahlungsquelle 5 emittierten Strahlung vor Reflektion an der Innenseite des Gehäuses 4 ist.

Ferner ist in Fig. 1 dargestellt, dass die Strahlungsquelle 5 mit einer Halteeinrichtung 9 an der Innenseite 6 des Gehäuses 4 befestigt ist. Die Halteeinrichtung 9 kann in diesem Zusammenhang eine Mehrzahl von Haltemitteln 10 aufweisen. Die Haltemittel 10 sind im dargestellten Ausführungsbeispiel als strebenförmige Halterungen ausgebildet. In dem in Fig. 1 dargestellten Ausführungsbeispiel ist gezeigt, dass die Stirnseite bzw. der stirnseitige Bereich der Strahlungsquelle 5 mit wenigstens zwei, insbesondere drei, Haltemitteln 10 der Halteeinrichtung 9 an der Innenseite 6 befestigt werden kann.

Insbesondere kann die Strahlungsquelle 5 drehfest über die Halteeinrichtung 9 an dem Gehäuse 4 fixiert sein. Dabei kann die Halteeinrichtung 9 weiter derart ausgebildet sein, dass das erzeugte Strahlungsbild bzw. Interferenzmuster in der Behandlungskammer 8 möglichst wenig beeinträchtigt wird.

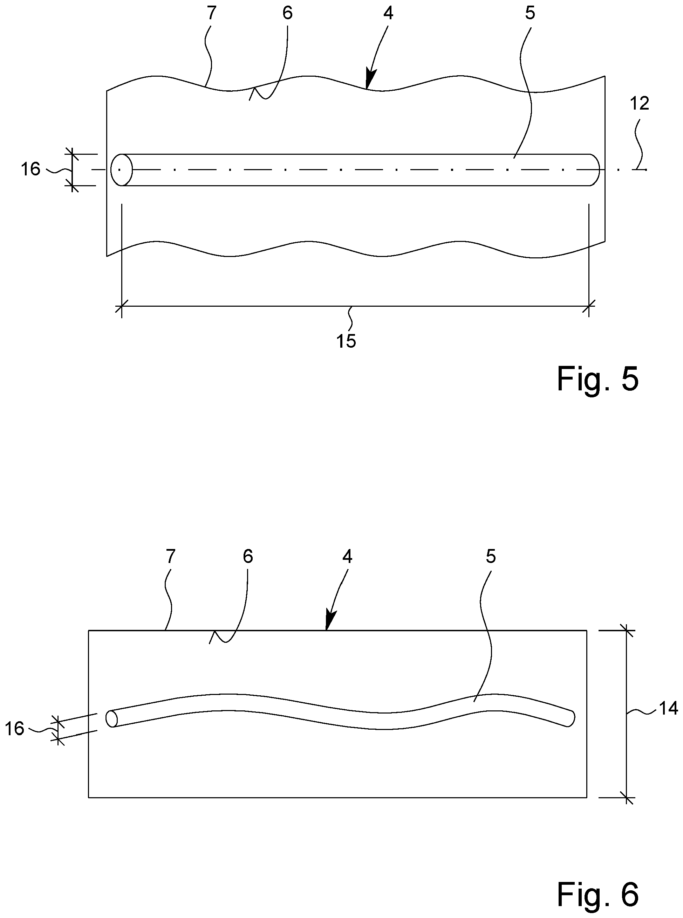

Die Innenseite 6 weist bei dem in Fig. 5 dargestellten Ausführungsbeispiel einen Reflexionsgrad von wenigstens 0,7 auf. In weiteren Ausführungsformen kann der Reflexionsgrad wenigstens 0,8, insbesondere wenigstens 0,9, betragen. Der Reflexionsgrad gibt letztlich denjenigen Anteil der von der Strahlungsquelle 5 emittierten Strahlung an, der an der Innenseite 6 reflektiert wird.

Wenngleich es nicht dargestellt ist, kann bei weiteren Ausführungsformen vorgesehen sein, dass die Innenseite 6 des Gehäuses 4 und/oder die Innenwandung 7 des Gehäuses 4 mit einer UV-Strahlung reflektierenden Beschichtung zumindest bereichsweise, insbesondere vollständig, beschichtet ist. Die Innenwandung 7 kann dabei als entnehmbare Komponente oder als Reflektor ausgebildet sein.

Des Weiteren ist nicht dargestellt, dass die Innenwandung 7 und/oder der Innenseite 6 als Material Metall aufweisen kann und/oder daraus bestehen kann. In weiteren Ausführungsformen kann die Innenwandung 7 ein Blech, insbesondere ein Aluminiumblech und/oder ein verzinktes Blech und/oder ein Edelstahlblech, sein.

Fig. 5 zeigt, dass die Innenwandung 7 des Gehäuses 4 gewellt ausgebildet ist. Letztlich kann die Innenwandung 7 bei dem in Fig. 5 dargestellten Ausführungsbeispiel eine derartige Formgebung aufweisen, dass die konstruktive Interferenz in der Behandlungskammer 8 erhöht wird.

Bei dem in Fig. 6 dargestellten Ausführungsbeispiel ist die Innenwandung 7 des Gehäuses 4 bzw. auch die Innenseite 6 des Gehäuses 4 eben - also flach und ohne Erhebungen - ausgebildet.

Fig. 1 zeigt, dass die Innenwandung 7 des Gehäuses 4 zylinderförmig ausgebildet ist.

Letztlich kann die Innenwandung 7 und/oder der Innenseite 6 in weiteren Ausführungsformen auch eine Mehrzahl von Erhebungen und/oder Vertiefungen aufweisen.

Die in Fig. 1 dargestellte Innenwandung 7 ist ferner rotationssymmetrisch ausgebildet. Es versteht sich jedoch, dass die Innenwandung 7 nicht zwingend symmetrisch ausgebildet sein muss.

Aus den Fig. 2 bis 4 geht anschaulich hervor, dass die Strahlungsquelle 5 derart in dem Gehäuse 4 bzw. in der Behandlungskammer 8 positioniert ist, dass sich keine symmetrische Anordnung der Strahlungsquelle 5 in dem Gehäuse 4 ergibt.

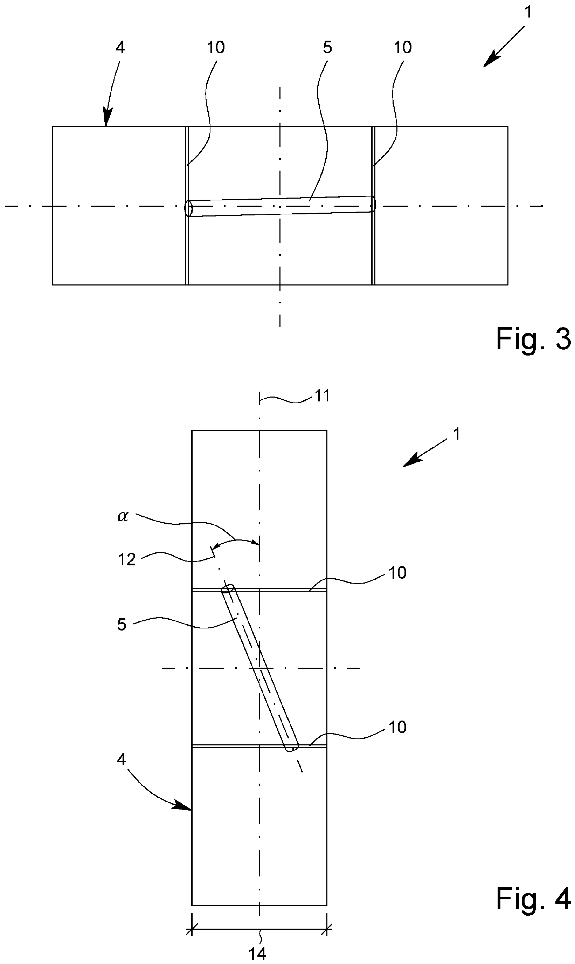

In Fig. 4 ist dargestellt, dass die Längsachse 11 des Gehäuses 4 versetzt zu der Längsachse 12 der Strahlungsquelle 5 ausgerichtet ist. Die Längsachse 11 des Gehäuses 4 ist in Richtung der größten Erstreckung angeordnet. Im dargestellten Ausführungsbeispiel entspricht die Längsachse 11 auch der Symmetrieachse des Gehäuses 4. Die Längsachse 12 der Strahlungsquelle 5 ist ebenfalls in Längserstreckung der Strahlungsquelle 5 angeordnet. Im dargestellten Ausführungsbeispiel sind die Längsachsen 11 und 12 zueinander schräg ausgerichtet. Sie können einen Winkel $\alpha$ zwischen 3° bis 30° einschließen, wie ebenfalls die Fig. 4 verdeutlicht.

Fig. 3 zeigt, dass sich in der Draufsicht keine Verschiebung der Achsen der Strahlungsquelle 5 und des Gehäuses 4 ergibt. Bei dem in den Fig. 1 bis 4 dargestellten Ausführungsbeispiel ist die Strahlungsquelle 5 letztlich relativ zum Gehäuse 4 in einer Ebene verschoben.

Fig. 1 zeigt, dass die Strahlungsquelle 5 dezentral in Bezug zum Gehäuse 4 angeordnet ist.

Die Halteeinrichtung 9 ist derart ausgebildet, dass die schräge Anordnung der Längsachsen 11 und 12 zueinander sichergestellt werden kann. In weiteren Ausführungsformen kann vorgesehen sein, dass eine Einstellung bzw. Veränderung der Schrägstellung der Strahlungsquelle 5 über eine Verstellung der Halteeinrichtung 9 erfolgen kann. Die Halteeinrichtung 9 kann demzufolge in weiteren Ausführungsformen einstellbar bzw. zur Verstellung der Strahlungsquelle 5 ausgebildet sein.

Die Schrägstellung der Strahlungsquelle 5 kann so ausgewählt sein, dass die gewünschten Interferenzeffekte durch konstruktive Interferenz erreicht werden können.

Des Weiteren zeigt die Fig. 4, dass die Längsachse 11 des Gehäuses 4 nicht koaxial zur Längsachse 12 der Strahlungsquelle 5 verläuft. Des Weiteren sind die Längsachsen 11 und 12 auch nicht parallel zueinander.

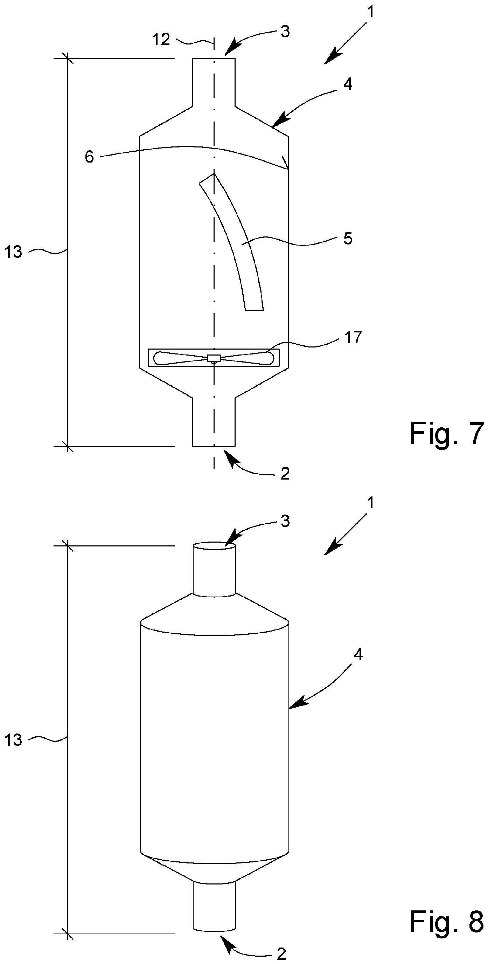

Das Gehäuse 4 kann eine Länge 13 zwischen 30 bis 200 cm aufweisen. Die Länge 13 ist schematisch in den Fig. 7 und 8 dargestellt. Das Gehäuse 4 kann einen Durchmesser 14, insbesondere einen Innendurchmesser, zwischen 8 bis 30 cm aufweisen.

Nicht dargestellt ist, dass die Strahlungsquelle 5 eine Mehrzahl von Leuchtmitteln, vorzugsweise LEDs, aufweisen kann. Dabei können die mehreren Leuchtmittel der Strahlungsquelle 5 in einem "array", insbesondere unmittelbar nebeneinander, angeordnet sein, so dass sich insbesondere eine langgestreckte Form der Strahlungsquelle 5 ergibt.

In dem dargestellten Ausführungsbeispiel ist vorgesehen, dass die Strahlungsquelle 5 eine langgestreckte und stabförmige Form aufweist.

Die Länge 15 der Strahlungsquelle 5 kann zwischen 5 cm bis 30 cm liegen.

Der Durchmesser 16 der Strahlungsquelle 5 kann in weiteren Ausführungsformen zwischen 1 bis 20 cm liegen, insbesondere 5 cm +/- 1 cm betragen.

Nicht dargestellt ist ferner, dass eine Mehrzahl von Strahlungsquellen 5 in dem Gehäuse 4 angeordnet ist. Die von den Strahlungsquellen 5 emittierten Strahlungen, insbesondere die UV-C-Strahlung, können sich demzufolge gegenseitig beeinflussen bzw. miteinander wechselwirken. Durch die mehreren Strahlungsquellen 5 kann letztlich die Leistung der bereitgestellten UV-Strahlung erhöht werden. Die Strahlungsquellen 5 können derart zueinander (relativ) angeordnet werden, dass die geforderten Interferenzeigenschaften erreicht werden können. So können in der Behandlungskammer 8 zwischen 2 bis 10 Strahlungsquellen 5 angeordnet sein.

Zudem ist nicht dargestellt, dass bei einer Mehrzahl von Strahlungsquellen 5 diese zumindest bereichsweise überlappend in dem Gehäuse 4 angeordnet sind. Die Länge des Überlappungsbereichs kann dabei zwischen 30 bis 80 % der Länge 15 wenigstens einer Strahlungsquelle 5 entsprechen. Auch können die Strahlungsquellen 5 zueinander windschief und/oder schräg und/oder versetzt angeordnet sein, insbesondere sind die Längsachsen 12 der Strahlungsquellen 5 zueinander versetzt und/oder schräg angeordnet. Die Anordnung der Strahlungsquellen 5 kann im Hinblick auf das erzeugte Interferenzbild erfolgen.

Fig. 7 zeigt, dass die Bestrahlungsvorrichtung 1 einen Ventilator 17 zum Erzeugen eines Mediumstromes vom Einlass 2 zum Auslass 3 umfasst. Im dargestellten Ausführungsbeispiel ist der Ventilator 17 hinter dem Einlass 2 angeordnet. In weiteren Ausführungsformen kann der Ventilator 17 auch vor dem Einlass 2 angeordnet sein.

Die von der Strahlungsquelle 5 bereitgestellte UV-Strahlung kann in einem Wellenlängenbereich von wenigstens 240 nm bis 300 nm, insbesondere in einem Wellenlängenbereich von 250 bis 285 nm und insbesondere bei 254 nm +/- 10% und/oder bei 278 nm +/- 10 % liegen.

Darüber hinaus ist nicht dargestellt, dass vor dem Einlass 2 ein Vorfilter angeordnet sein kann. Der Vorfilter kann derart ausgebildet sein, dass Partikel mit einem Durchmesser von größer 1 $\mu$m, bevorzugt größer 0,5 $\mu$m, aus dem Mediumstrom gefiltert werden.

Der Mediumstrom kann eine Strömungsgeschwindigkeit zwischen 1 bis 2 m/s, insbesondere von 1,7 m/s +/- 20 %, aufweisen.

In weiteren Ausführungsformen kann die Strahlungsquelle 5 eine Leistung zwischen 50 bis 500 W, bevorzugt zwischen 80 bis 200 W, aufweisen. Dabei können insbesondere wenigstens 10 %, bevorzugt zwischen 20 % bis 50 %, weiter bevorzugt zumindest im Wesentlichen zwischen 30 % bis 40 %, auf den Anteil der Leistung der Strahlungsquelle 5 für die bereitgestellte UV-C-Strahlung entfallen.

Die Strömung des Mediumstromes in der Behandlungskammer 8 kann laminar und/oder turbulent ausgebildet sein. Letztlich kann die Strömung auch durch den Ventilator 17 beeinflusst werden, der für die Durchströmung des Gehäuses 4 sorgt.

**Ausführungsbeispiel:**

Bei durchgeführten Versuchen hat sich gezeigt, dass bei einer Bestrahlungsvorrichtung 1 mit einer Leistung von 190 W +/- 10 % und einer Intensität an der Oberfläche der Strahlungsquelle 5 von etwa 4233 W/m$^2$ +/- 10 % in Abhängigkeit von verschiedenen Durchströmungsgeschwindigkeiten des Mediumstromes die nachfolgend angegebenen Abtötungsraten der Mikroorganismen erreicht werden können. Die Abtötungsrate der Mikroorganismen ist auch von dem Widerstand der jeweiligen Mikroorganismen gegen UV-C-Strahlung und von der jeweiligen Verweilzeit in dem Gehäuse 4 abhängig.

Das Gehäuse 4 hat eine Länge von 100 cm bei einem Innendurchmesser von 15 cm. Die Strahlungsquelle 5 hat einen Durchmesser von circa 5 cm +/- 10 % bei einer Länge von 15 cm. Die verwendete Strahlungsquelle 5 hat UV-Strahlung bei einer Wellenlänge von 254 nm +/- 2 % bereitgestellt.

Als Medium ist mit Mikroorganismen belastete Luft gewählt worden.

Die nachfolgend angegebene Tabelle stellt die Versuchsergebnisse der erreichten Abtötungsrate für verschiedene Mikroorganismen dar.

| Volumenstrom | Viren | Bakterien | Pilze |
|---|---|---|---|
| 100 m$^3$/h | > 99,999 % | > 99,999 % | 98,72 % |
| 200 m$^3$/h | > 99,999 % | > 99,999 % | 88,68 % |
| 300 m$^3$/h | > 99,999 % | 99,997 % | 76,60 % |
| 400 m$^3$/h | > 99,999 % | 99,96 % | 66,36 % |
| 500 m$^3$/h | > 99,999 % | 99,82 % | 58,17 % |
| 600 m$^3$/h | > 99,999 % | 99,49 % | 51,53 % |
| 700 m$^3$/h | > 99,999 % | 98,92 % | 46,34 % |
| 800 m$^3$/h | 99,999 % | 98,10 % | 41,99 % |
| 900 m$^3$/h | 99,998 % | 97,04 % | 38,38 % |
| 1.000 m$^3$/h | 99,995 % | 95,79 % | 35,32 % |

Die vorgenannte Tabelle verdeutlicht die besonders effiziente Viren- und Bakterieninaktivierung - selbst bei hohen Volumenströmen. Die vorgenannte Tabelle zeigt ferner, dass durch die erfindungsgemäße Ausbildung der Bestrahlungsvorrichtung 1 nicht nur effizient Viren und Bakterien sondern auch Pilze abgetötet werden können.

**Bezugszeichenliste:**

| | |
|---|---|
| 1 | Bestrahlungsvorrichtung |
| 2 | Einlass |
| 3 | Auslass |
| 4 | Gehäuse |
| 5 | Strahlungsquelle |
| 6 | Innenseite |
| 7 | Innenwandung |
| 8 | Behandlungskammer |
| 9 | Halteeinrichtung |
| 10 | Haltemittel |
| 11 | Längsachse von 4 |
| 12 | Längsachse von 5 |
| 13 | Länge von 4 |
| 14 | Durchmesser von 4 |
| 15 | Länge von 5 |
| 16 | Durchmesser von 5 |
| 17 | Ventilator |
| $\alpha$ | Winkel |

**Patentansprüche**

1. Bestrahlungsvorrichtung (1) zur UV-Bestrahlung, insbesondere UV-C-Bestrahlung, eines die Bestrahlungsvorrichtung (1) durchströmenden Mediums, insbesondere Wasser oder Luft, insbesondere zur Inaktivierung von in dem Medium befindlichen Mikroorganismen, wie Bakterien, Keime, Schimmel und/oder Viren, mit einem einen Einlass (2) und einen Auslass (3) für das Medium aufweisenden zu durchströmenden Gehäuse (4) und wenigstens einer im Inneren des Gehäuses (4) angeordneten, UV-Strahlung emittierenden Strahlungsquelle (5) zur Bestrahlung des das Gehäuse (4) durchströmenden Mediums,
wobei das Gehäuse (4) an der der Strahlungsquelle (5) zugewandten Innenseite (6) zumindest bereichsweise, vorzugsweise vollflächig, reflektierend mit einem Reflexionsgrad für die von der Strahlungsquelle (5) emittierten UV-Strahlung von wenigstens 0,6 ausgebildet ist,
wobei die Strahlungsquelle (5) derart in dem Gehäuse (4) angeordnet ist, dass die von der Strahlungsquelle (5) emittierte Strahlung an der Innenseite (6) des Gehäuses (4) reflektiert wird und dass die von der Strahlungsquelle (5) emittierte Strahlung mit der reflektierten Strahlung konstruktiv interferiert und/oder
wobei die Strahlungsquelle (5) derart in dem Gehäuse (4) angeordnet ist, dass die von der Strahlungsquelle (5) emittierte Strahlung an der Innenseite (6) des Gehäuses (4) reflektiert wird und dass die von der Strahlungsquelle (5) emittierte Strahlung zu der reflektierten Strahlung einen von einem ganzzahligen Vielfachen der halben Wellenlänge und/oder von der halben Wellenlänge unterschiedlichen Gangunterschied aufweist und/oder dass die von der Strahlungsquelle (5) emittierte Strahlung mit der reflektierten Strahlung zumindest im Wesentlichen nicht destruktiv interferiert, insbesondere wobei weniger als 30 %, bevorzugt weniger als 25 % und insbesondere zwischen 0 % bis 20 %, der von der Strahlungsquelle (5) emittierten Strahlung destruktiv interferiert.

2. Bestrahlungsvorrichtung nach Anspruch 1, **dadurch gekennzeichnet, dass** die Strahlungsquelle (5) derart in dem Gehäuse (4) angeordnet ist, dass in dem durch Wechselwirkung der von der Strahlungsquelle (5) emittierten Strahlung mit der reflektierten Strahlung erzeugten Interferenzbild der Anteil der konstruktiven Interferenz den Anteil der destruktiven Interferenz übersteigt, vorzugsweise um wenigstens 10 %, bevorzugt um wenigstens 20 %, weiter bevorzugt um wenigstens 40 %, vorzugsweise um wenigstens 50 %, insbesondere um wenigstens 70 %, und/oder dass die Strahlungsquelle (5) derart in dem Gehäuse (4) angeordnet ist, dass der Anteil der wechselwirkenden und konstruktiv interferierenden Strahlung den Anteil der wechselwirkenden und destruktiv interferierenden Strahlung übersteigt, vorzugsweise um wenigstens 10 %, bevorzugt um wenigstens 20 %, weiter bevorzugt um wenigstens 40 %, vorzugsweise um wenigstens 50 %, insbesondere um wenigstens 70 %.

3. Bestrahlungsvorrichtung nach Anspruch 1 oder 2, **dadurch gekennzeichnet, dass** die Strahlungsquelle (5) derart in dem Gehäuse (4) angeordnet ist, dass das durch Wechselwirkung der von der Strahlungsquelle (5) emittierten Strahlung mit der reflektierten Strahlung erzeugte Interferenzbild eine, insbesondere gemittelte, maximale Gesamt-Intensität des Interferenzbildes aufweist, die um wenigstens 50 %, bevorzugt wenigstens 100 %, weiter bevorzugt wenigstens 200 %, vorzugsweise wenigstens 280 %, weiter bevorzugt zwischen 300 % bis 500 %, größer als die maximale Intensität der direkten, von der Strahlungsquelle (5) emittierten Strahlung vor Reflektion an der Innenseite des Gehäuses (4) ist.

4. Bestrahlungsvorrichtung nach einem der vorhergehenden Ansprüche , **dadurch gekennzeichnet, dass** die Innenseite (6) des Gehäuses (4) einen Reflexionsgrad für die von der Strahlungsquelle (5) emittierte Strahlung von wenigstens 0,7, bevorzugt wenigstens 0,8, weiter bevorzugt wenigstens 0,9, aufweist und/oder dass die Innenseite (6), insbesondere eine Innenwandung (7), des Gehäuses (4) mit einer UV-Strahlung reflektierenden Beschichtung zumindest bereichsweise, vorzugswese vollständig, beschichtet ist und/oder dass die Innenwandung (7) und/oder die Innenseite (6) des Gehäuses (4) als Material Metall aufweist und/oder daraus besteht, insbesondere wobei die Innenwandung (7) und/oder die Innenseite (6) ein Blech, insbesondere Aluminium aufweisendes Blech und/oder ein verzinktes Blech und/oder ein Edelstahl-Blech, aufweist und/oder daraus besteht.

5. Bestrahlungsvorrichtung nach einem der vorhergehenden Ansprüche, **dadurch gekennzeichnet, dass** die Innenwandung (7) und/oder die Innenseite (6) des Gehäuses (4) zylinderförmig und/oder gewellt oder eben ausgebildet ist und/oder dass die Innenwandung (7) und/oder die Innenseite (6) des Gehäuses (4) eine Mehrzahl von Erhebungen und/oder Vertiefungen aufweist und/oder dass die Innenwandung (7) des Gehäuses (4) rotationssymmetrisch ausgebildet ist.

6. Bestrahlungsvorrichtung nach einem der vorhergehenden Ansprüche, **dadurch gekennzeichnet, dass** die Längsachse (11) des Gehäuses (4), insbesondere der Innenwandung (7) des Gehäuses (4), versetzt, vorzugsweise schräg, zu der Längsachse (12) der Strahlungsquelle (5) ausgerichtet ist, insbesondere wobei die Längsachse (11) des Gehäuses (4), insbesondere der Innenwandung (7) des Gehäuses (4), einen Winkel (a) größer 2°, bevorzugt zwischen 2° bis 50°, weiter bevorzugt zwischen 3° bis 15°, zu der Längsachse (12) der Strahlungsquelle (5) einschließt,
und/oder dass die Strahlungsquelle (5) dezentral in Bezug zum Gehäuse (4) angeordnet ist.

7. Bestrahlungsvorrichtung nach einem der vorhergehenden Ansprüche, **dadurch gekennzeichnet, dass** das Gehäuse (4) eine Länge (13) zwischen 30 bis 200 cm, bevorzugt zwischen 80 bis 150 cm, aufweist und/oder dass das Gehäuse (4) einen Durchmesser (14), vorzugsweise einen Innendurchmesser, zwischen 8 bis 30 cm, bevorzugt zwischen 10 bis 20 cm, aufweist.

8. Bestrahlungsvorrichtung nach einem der vorhergehenden Ansprüche, **dadurch gekennzeichnet, dass** die Strahlungsquelle (5) eine Mehrzahl von Leuchtmitteln, vorzugsweise LEDs, aufweist und/oder dass die Strahlungsquelle (5) eine zumindest im Wesentlichen langgestreckte und/oder stabförmige Form aufweist, insbesondere wobei die Strahlungsquelle (5) eine Länge (13) von wenigstens 5 cm, bevorzugt zwischen 5 cm bis 30 cm, weiter bevorzugt zwischen 10 cm bis 20 cm, aufweist.

9. Bestrahlungsvorrichtung nach einem der vorhergehenden Ansprüche, **dadurch gekennzeichnet, dass** die Strahlungsquelle (5) einen Durchmesser (14) von wenigstens 1 cm, bevorzugt zwischen 1 cm und 20 cm, weiter bevorzugt zwischen 2 cm und 10 cm und insbesondere von 5 cm +/- 1 cm, aufweist.

10. Bestrahlungsvorrichtung nach einem der vorhergehenden Ansprüche, **dadurch gekennzeichnet, dass** eine Mehrzahl von Strahlungsquellen (5) in dem Gehäuse (4) angeordnet ist, insbesondere wobei zwischen 2 bis 10, bevorzugt zwischen 2 bis 5, Strahlungsquellen (5) in dem Gehäuse (4) angeordnet sind.

11. Bestrahlungsvorrichtung nach einem der vorhergehenden Ansprüche, **dadurch gekennzeichnet, dass** die mehreren Strahlungsquellen (5) zumindest bereichsweise überlappend in dem Gehäuse (4) angeordnet sind, insbesondere wobei die Länge des Überlappungsbereiches wenigstens 20 %, bevorzugt wenigstens 30 %, weiter bevorzugt zwischen 30 % bis 80 %, der Länge (13) wenigstens einer Strahlungsquelle (5) entspricht.

**12.** Bestrahlungsvorrichtung nach einem der vorhergehenden Ansprüche, **dadurch gekennzeichnet, dass** die Strahlungsquellen (5) zueinander windschief angeordnet sind und/oder dass die Längsachsen (12) der Strahlungsquellen (5) zueinander versetzt, vorzugsweise schräg, angeordnet sind.

**13.** Bestrahlungsvorrichtung nach einem der vorhergehenden Ansprüche, **dadurch gekennzeichnet, dass** die Vorrichtung einen Ventilator (17) zum Erzeugen des Mediumstromes vom Einlass (2) zum Auslass (3) umfasst.

**14.** Bestrahlungsvorrichtung nach einem der vorhergehenden Ansprüche, **dadurch gekennzeichnet, dass** die mindestens eine Strahlungsquelle (5) UV-Strahlung in einem Wellenlängenbereich von wenigstens 240 nm bis 300 nm, bevorzugt in einem Wellenlängenbereich von 250 nm bis 285 nm, weiter bevorzugt von 270 nm bis 280 nm und insbesondere von 254 nm +/- 10 % und/oder von 278 nm +/- 10 %, emittiert.

**15.** Bestrahlungsvorrichtung nach einem der vorhergehenden Ansprüche, **dadurch gekennzeichnet, dass** vor dem Einlass (2) ein Vorfilter angeordnet ist, insbesondere wobei der Vorfilter derart ausgebildet ist, dass Partikel mit einem Durchmesser größer 1 $\mu$m, bevorzugt größer 0,5 $\mu$m, aus dem Mediumstrom gefiltert werden.

Fig. 1

Fig. 2

Fig. 3

Fig. 4

Fig. 5

Fig. 6

Fig. 7

Fig. 8

Europäisches Patentamt
European Patent Office
Office européen des brevets

# EUROPÄISCHER RECHERCHENBERICHT

Nummer der Anmeldung

EP 20 18 1097

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2019/049129 A1 (HUANG JASON AN-CHENG [TW] ET AL) 14. Februar 2019 (2019-02-14) * Absätze [0013], [0024], [0025], [0027], [0029], [0030], [0035]; Anspruch 1; Abbildungen 2,3,4 * ----- | 1-15 | INV. A61L9/20 C02F1/32 F21V7/00 |
| X | DE 10 2017 117324 A1 (BLOMEIER MAXIMILIAN [DE]; MAIER PETER [DE]; WEBER MARKUS [DE]) 31. Januar 2019 (2019-01-31) * Absätze [0007] - [0009], [0027] - [0032]; Abbildungen 1-5 * ----- | 1-15 | |
| X | DE 200 21 236 U1 (LIFELIGHT GMBH [DE]) 8. März 2001 (2001-03-08) * Seite 3, Absatz 5 - Seite 4, Absatz 3; Abbildungen 1,2 * ----- | 1-15 | |
| X | EP 3 111 963 A1 (TROJAN TECH INC [CA]) 4. Januar 2017 (2017-01-04) * Clause 21; Spalte 21, Absatz 86; Abbildungen 1-7,8A,8B,12-16 * * Clauses 62,65, 66; Spalte 28 * ----- | 1-15 | |

| | | | |
|---|---|---|---|
| | | | RECHERCHIERTE SACHGEBIETE (IPC) |
| | | | A61L C02F F21V F21Y A23L |

Der vorliegende Recherchenbericht wurde für alle Patentansprüche erstellt

| Recherchenort | Abschlußdatum der Recherche | Prüfer |
|---|---|---|
| München | 9. November 2020 | Beckmann, Oliver |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 20 18 1097

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-11-2020

| Im Recherchenbericht angeführtes Patentdokument | Datum der Veröffentlichung | Mitglied(er) der Patentfamilie | Datum der Veröffentlichung |
|---|---|---|---|
| US 2019049129 A1 | 14-02-2019 | CN 107670086 A<br>DE 102017131097 A1<br>TW 201909930 A<br>US 2019049129 A1 | 09-02-2018<br>14-02-2019<br>16-03-2019<br>14-02-2019 |
| DE 102017117324 A1 | 31-01-2019 | DE 102017117324 A1<br>WO 2019025199 A1 | 31-01-2019<br>07-02-2019 |
| DE 20021236 U1 | 08-03-2001 | DE 20021236 U1<br>WO 0248619 A1 | 08-03-2001<br>20-06-2002 |
| EP 3111963 A1 | 04-01-2017 | CA 2559551 A1<br>EP 1737501 A1<br>EP 3111963 A1<br>US 2005263716 A1<br>US 2008292514 A1<br>US 2012248329 A1<br>US 2013277571 A1<br>US 2014360947 A1<br>WO 2005087277 A1 | 22-09-2005<br>03-01-2007<br>04-01-2017<br>01-12-2005<br>27-11-2008<br>04-10-2012<br>24-10-2013<br>11-12-2014<br>22-09-2005 |

EPO FORM P0461

Für nähere Einzelheiten zu diesem Anhang : siehe Amtsblatt des Europäischen Patentamts, Nr.12/82